# EUROPÄISCHE PATENTSCHRIFT

(11) **EP 0 765 654 B1**
(45) Veröffentlichungstag und Bekanntmachung des Hinweises auf die Patenterteilung: **05.12.2001**
(21) Anmeldenummer: 96115351.7
(22) Anmeldetag: 25.09.1996
(51) Int. Cl.: A61J 1/00, B32B 7/06

(54) **Medizinischer Beutel**
Medical bag
Poche médical

(30) Priorität: 29.09.1995 DE 19536546
(43) Veröffentlichungstag der Anmeldung: 02.04.1997
(73) Patentinhaber: Fresenius AG, 61350 Bad Homburg v.d.H (DE)
(72) Erfinder: Nicola, Thomas, 57350 Spicheren (FR); Kreischer, Thomas, 66113 Saarbrücken (DE)
(74) Vertreter: Luderschmidt, Schüler & Partner GbR

(56) Entgegenhaltungen:
- EP-A- 0 114 964
- EP-A- 0 345 774
- EP-A- 0 380 145
- EP-A- 0 437 856
- EP-A- 0 513 364
- EP-A- 0 619 998
- EP-A- 0 635 254
- WO-A-88/06897
- WO-A-93/17863
- DE-A- 3 919 360
- DE-A- 4 410 876
- FR-A- 2 523 847
- FR-A- 2 544 612
- US-A- 3 211 144

## Beschreibung

Die Erfindung betrifft einen medizinischen Beutel mit einer Kammer und einem rohrförmigen Anschlußstück.

Beutel aus thermoplastischen Materialien sind üblicherweise im leeren Zustand nicht heißsterilisierbar, da die Innenoberflächen des Beutels, die optimale Schweißeigenschaften aufweisen, sich häufig durch das Heißsterilisieren innig aneinanderlegen und sogar zusammenkleben. Bei der üblichen Heißdampfsterilisation bei einer Temperatur von etwa 121°C ist ein solches Aneinanderhaften der Innenoberflächen solcher Beutel also die Regel, so daß in diese Beutel nur noch wenig Flüssigkeit eingefüllt werden kann, es sei denn, daß die Flüssigkeit unter erhöhtem Druck der Beutelkammer zugeführt wird. Dennoch kann auch durch eine solche Druckbehandlung nicht sichergestellt werden, daß feste Klebestellen aufgelöst werden.

Um dem Verkleben an größeren Flächenbereichen entgegenzutreten, wurden Beutelfolien bisher mechanisch vorbehandelt. So wurden beispielsweise die Oberflächen aufgerauht oder aber es wurden erhabene Stellen in die Oberflächen eingearbeitet, beispielsweise punktförmige Erhebungen, Rillen oder Stege. Dies hatte zur Folge, daß die Innenoberflächen nur noch in einem geringen Oberflächenbereich aneinander haften, so daß diese Bereiche dann beim Füllen aufgelöst werden.

Die US-A 32 11 144 beugt beispielsweise der Kohäsion der Innenoberflächen dadurch vor, daß mindestens eine der Innenoberflächen durch Anwendung einer Rolle mit entsprechend ausgebildeter Oberfläche aufgerauht wird. Als eine weitere Möglichkeit zum Aufrauhen der Oberfläche werden Ätzen oder Sandstrahlen genannt, so daß es nicht mehr zu einem Ankleben der Innenoberflächen kommen soll.

Eine derartige Behandlung der Folienoberflächen ist jedoch sehr zeit- und kostenaufwendig.

Medizinische Beutel werden deshalb nachwievor vorwiegend auf die Weise hitzesterilisiert, indem man vor dem Sterilisieren mit Luft oder Flüssigkeit wie Wasser oder bereits mit der zur verwendenden medizinischen Flüssigkeit füllt und sodann die Hitzesterilisation vornimmt. Ein derartiges Verfahren wird auch in der EP-A1-0 114 964 offenbart. Dort werden medizinische Behälter beschrieben, die durch Verschweißen von Folien gebildet werden, welche aus einem Polymergemisch bestehen. Vor dem Sterilisieren werden die Behälter mit Luft, Wasser oder einer medizinischen Flüssigkeit gefüllt.

Aufgabe der Erfindung ist es, einen medizinischen Beutel zur Verfügung zu stellen, der nach dem Heißsterilisieren im leeren Zustand nicht innig aneinander liegende Innenoberflächen oder sogar zusammengeklebte Oberflächen aufweist, und zwar ohne daß die Beutelfolien mechanisch vorbehandelt oder mit erhabenen Stellen versehen worden wären. Aufgabe der Erfindung ist es ferner, sterilisierte medizinische Beutel zur Verfügung zu stellen, welche nach einer Hitzesterilisierung in ungefülltem Zustand direkt als Leerbeutel eingesetzt werden können.

Die Aufgabe wird durch den Gegenstand des Anspruchs 1 gelöst. Dieser betrifft einen ungefüllten hitzesterilisierten medizinischen Leerbeutel mit mindestens einer Kammer und mindestens einem rohrförmigen Anschluß, dessen Randbereich durchgeschweißt ist, erhältlich durch Hitzesterilisation des Leerbeutels, dadurch gekennzeichnet, daß die innere Folienoberfläche aus einem Matrix-Phasen-Polymer-System besteht, wobei die Innenoberflächen des Beutels nicht zusammenhaften, ohne daß die inneren Oberflächen mechanisch vorbehandelt oder mit erhabenen Stellen versehen werden. Vorteilhafte Ausführungsformen der Erfindung werden in den Patentansprüchen 2-6 beschrieben. Darüberhinaus offenbart Anspruch 7 ein Verfahren zur Herstellung eines hitzesterilisierten Leerbeutels.

Überraschenderweise wurde nun festgestellt, daß das in der am 05.10.1995 offengelegten DE 44 10 876 beschriebene Matrix-Phasen-Polymer-System zu einem medizinischen Beutel führt, der ohne weitere Vorbehandlung der Folien im leeren Zustand hitzesterilisierbar ist, wobei nach dem Sterilisieren die Innenoberflächen nicht aneinander haften.

Demzufolge kann ein solcher medizinischer Beutel vorteilhafterweise zu Drainage-Zwecken in der Urologie oder der Peritonealdialyse eingesetzt werden. Derartige Beutel müssen in aller Regel sterilisiert sein, da sie über Schlauchleitungen bzw. Katheter mit dem Körper des Patienten verbunden werden, so daß eventuelle Kontaminationen des Beutels gesundheitsgefährdend sein können.

Zur Herstellung des Beutels werden vorzugsweise sogenannte Blas- oder auch Schlauchfolien verwendet. Diese können gegebenenfalls gefaltet werden und müssen lediglich an zwei Seiten des Außenrandes, nämlich den beiden Öffnungen, verschlossen, d.h. verschweißt werden. Es ist aber auch die Verwendung von einzelnen Folienblättern denkbar, die aber umlaufend verschweißt werden müssen.

Die Folie weist zumindest in der inneren Schicht, welche auch die Siegelschicht ist, im wesentlichen zwei Komponenten auf, nämlich ein Matrix-Polymer und ein Phasen-Polymer. Das System aus Matrix- und Phasen-Polymer wird im folgenden als Matrix-Phasen-Polymer-System bezeichnet.

Als Matrix-Polymere können Polymere mit hohem Anregungs- bzw. Schmelztemperaturbereich, wie z.B. Polyethylen-Homopolymer, Polypropylen-Homopolymer und Polypropylen-Copolymer verwendet werden. Polyethylen wird dabei als High-Density-Polyethylen (HDPE) eingesetzt. Von den genannten Matrix-Polymeren ist das Polypropylen-Copolymer bevorzugt. Besonders bevorzugt ist ein Polypropylen-Random-Copolymer.

Als Phasen-Polymere können nur Polymere mit ebenfalls hohem Anregungsbereich, wie z.B. Styrol-Ethylen/Butylen-Styrol-Block-Polymer (SEBS), Styrol-Ethylen/Propylen-Styrol-Block-Polymer (SEPS) und/oder Styrol-Isopren-Styrol-Triblock-Polymer (SIS) verwendet werden. Vorzugsweise wird Styrol-Ethylen-Butylen-Block-Copolymer eingesetzt. Der Anteil des PhasenPolymers in der Polymerschicht sollte dabei im Bereich von 1 bis 40 Gew.-%, bezogen auf das gesamte Matrix-Phasen-Polymer-System, liegen. Als Matrix-Polymer-System ist bevorzugt, daß als Matrixpolymer Polypropylen enthält. Dieses Matrix-Polymer liegt vorteilhafterweise vor in einer Menge von 60-95 Gewichts-%. Weiter bevorzugt ist als Phasen-Polymer Styrol-Ethylen-Butylen-Styrol-Block-Copolymer (SEBS) oder Styrol-Ethylen-Propylen-Styrol-Block-Copolymer (SEPS), die in einer Menge von 2-40 Gewichts-% vorliegen. Vorteilhafterweise weisen diese Phasenpolymere ein Molekulargewicht über 100 000 g/mol auf und besitzen keine wesentlichen Diblockanteile.

Die Schweißnaht des Außenrandbereiches weist mindestens einen Stutzen als Anschlußstück in mindestens einer Kammer auf, wobei dieser Stutzen ein Einlaßstutzen ist.

Der Beutel kann auch eine Aufhängevorrichtung und/oder Zuspeitzstelle enthalten.

Das Verfahren zur Herstellung des erfindungsgemäßen medizinischen Leerkammerbeutels ist dadurch gekennzeichnet, daß die Verschweißung im Außenrandbereich bei einer solchen Temperatur durchgeführt wird, die kein Aufreißen des verschweißten Bereichs ohne Zerstörung des Beutels mehr zuläßt.

Die Dauer des Verschweißvorganges liegt vorzugsweise im Bereich von 1 bis 8 Sekunden und die während des Schweißvorganges auf die zu verschweißenden Bereiche ausgeübte Flächenpressung liegt vorzugsweise im Bereich von 0,1 bis 3 N/mm². Für die beiden genannten Parameter sind jedoch auch Werte außerhalb der genannten Bereiche möglich. Die beiden Parameter sind somit nicht auf die bevorzugten Bereiche beschränkt.

Weitere Einzelheiten, Merkmale und Vorteile der Erfindung ergeben sich aus der nachfolgenden Beschreibung eines Ausführungsbeispiels anhand der Zeichnungen.

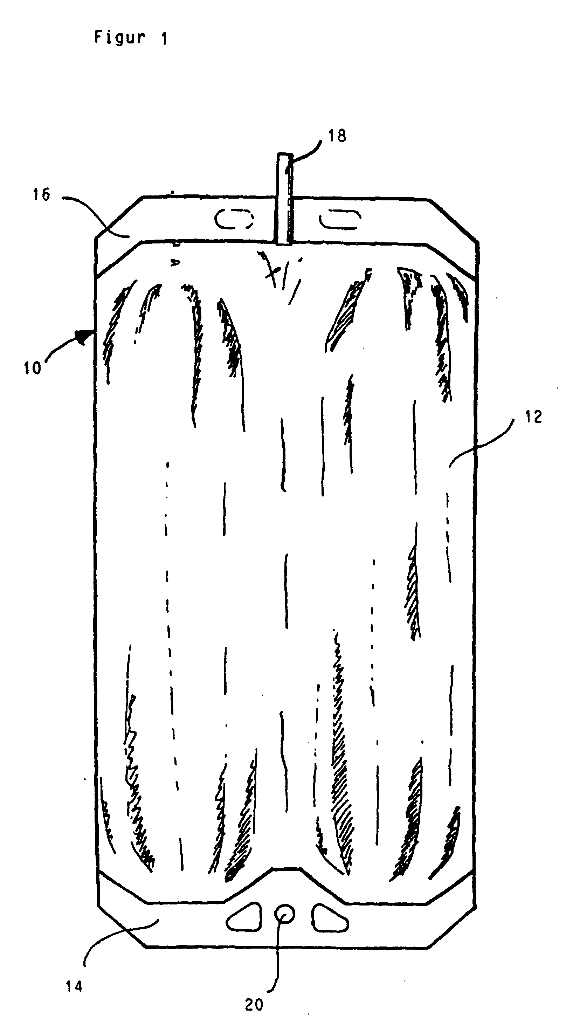

Es zeigt
- Figur 1: eine schematisch vereinfachte Darstellung eines erfindungsgemäßen Beutels.

In Fig. 1 ist ein Beutel 10 mit einer Kammer 12 gezeigt.

Dieser Beutel 10 ist aus einer Blasfolie gebildet, so daß lediglich der obere Randbereich 14 und der untere Randbereich 16 durchgeschweißt sind. In den Randbereich 16 ist dabei ein Anschlußstutzen 18 eingeschweißt, der rohrförmig ausgebildet ist und eine Strömungsverbindung mit der Kammer 12 schafft. Dieses Anschlußstück 18 kann mit einem nicht gezeigten Schlauch oder sonstigem Anschlußstück verbunden sein.

Der obere Rand verfügt im übrigen über eine Aufhängeöffnung 20, so daß der Beutel 10 im Bedarfsfall aufgehängt werden kann.

Die Beutelfolie wird auf einer Koextrusions-Blasfolienanlage hergestellt.

Es wird folgende Blasfolie hergestellt:

Es werden 85 Gewichts-% PP-Homopolymer mit einem Erweichungspunkt oberhalb 121° C (Novolen 3200 HX der BASF) mit 15 Gewichts-% SEBS (Kraton G 1650 der Firma Shell) compoundiert und anschließend auf der Blasfolienanlage zu einer Blasfolie verarbeitet.

Danach wird die Blasfolie entsprechend der Länge der Beutel geschnitten und weiter zu einem Beutel 10 konfektioniert, wie er in Fig. 1 gezeigt ist. Dabei werden die Schweißbereiche 14 und 16 bei ca. 140° C durchgeschweißt.

Füllt man einen derartigen Beutel mit 3-41 Wasser von Raumtemperatur und läßt diesen aus 2 m Höhe fallen, so zerplatzt dieser Beutel nicht. Gleichermaßen erfüllt er die Transparenz zur Kontrolle des Füllguts.

Solange diese Eigenschaften erfüllt sind, ist der eigentliche Folienaufbau variabel und die geforderte Eigenschaft des Heißsterilisierens ohne Haft- bzw. Klebeneigung lediglich von dem Einsatz des Phasenmatrix-Polymersystems abhängig.

Die Steifigkeit und Festigkeit der Folie ist abhängig von ihrer Dicke. Sie liegt üblicherweise in einem Bereich von 0,1-0,3 mm und kann im Bedarfsfall auch außerhalb dieser Grenzen liegen.

Ein derart hergestellter Beutel 10 eignet sich überraschenderweise auch gut zum Einbau in ein CAPD-Schlauchsystem als Leerbeutel. Er ist über eine Schlauchleitung an einen Peritonealdialysekatheter eines Patienten angeschlossen und muß aus diesen Gründen absolut steril sein. Infolgedessen wird das gesamte System in einem Umbeutel untergebracht, und anschließend in einer Heißsterilisationsanlage bei 121° C auf übliche Weise heißsterilisiert.

## Patentansprüche

1. Ungefüllter hitzesterilisierter medizinischer Leerbeutel (10) mit mindestens einer Kammer (12) und mindestens einem rohrförmigen Anschluß (18), dessen Randbereich (14,16) durchgeschweißt ist, erhältlich durch Hitzesterilisation des Leerbeutels, **dadurch gekennzeichnet, daß** die innere Folienoberfläche aus einem Matrix-Phasen-Polymer-System besteht, wobei die Innenoberflächen des Beutels nicht zusammenhaften, ohne daß die inneren Oberflächen mechanisch vorbehandelt oder mit erhabenen Stellen versehen werden.

2. Beutel gemäß Anspruch 1, **dadurch gekennzeichnet, daß** das Matrixpolymer des Matrix-Phasen-Polymer-Systems ein Polyethylen-Homopolymer, ein Polypropylen-Homopolymer oder ein Polypropylen-Copolymer ist.

3. Beutel gemäß Anspruch 2, **dadurch gekennzeichnet, daß** das Matrixpolymer ein Polypropylen-Copolymer, vorzugsweise ein Polypropylen-Random-Copolymer, ist.

4. Beutel gemäß Anspruch 1, **dadurch gekennzeichnet, daß** das Phasenpolymer des Matrix-Phasen-Polymer-Systems ein Styrol-Ethylen/Butylen-Styrol-Triblock-Polymer (SEBS), ein Styrol-Ethylen/Butylen-Styrol-Triblock-Polymer (SEBS) mit einem Styrol-Ethylen/Butylen-Diblock-Anteil (SEB), ein Styrol-Ethylen/Propylen-Styrol-Triblock-Polymer (SEPS), ein Styrol-Butadien-Styrol-Triblock-Polymer ((SBS) und/oder ein Stryrol-Isopren-Styrol-Triblock-Polymer (SIS) und/oder ein Ethylen-α-Olefin-Copolymer ist.

5. Beutel gemäß einem der Ansprüche 1 bis 4, **dadurch gekennzeichnet, daß** der Anteil des Phasenpolymers in dem Polymersystem im Bereich von 1 - 40, vorzugsweise 10 - 20 Gew.-%, bezogen auf das Matrix-Phasen-Polymer-System liegt.

6. Beutel gemäß einem der Ansprüche 1 bis 4, **dadurch gekennzeichnet, daß** die Folie mehrschichtig und coextrudiert ist.

7. Verfahren zur Hersteilung eines ungefüllten hitzesterilisierten medizinischen Leerbeutels gemäß den Ansprüchen 1 - 6, **dadurch gekennzeichnet, daß** die Verschweißung im Außenrandbereich (14, 16) bei einer Temperatur durchgeführt wird, die kein Aufreißen des verschweißten Bereichs ohne Zerstörung des Beutels zuläßt.

## Claims

1. An unfilled heat-sterilised empty medical bag (10) with at least one chamber (12) and at least one tubular connection (18), the marginal portion (14, 16) of which is welded through, obtainable by heat sterilisation of the empty bag, **characterised in that** the inner surface of the film consists of a matrix-phase-polymer-system, the inner surfaces of the bag not clinging together without the inner surfaces having been mechanically preheated or provided with raised areas.

2. A bag according to claim 1, **characterised in that** the matrix polymer of the matrix-phase-polymer-system is a polyethylene homopolymer, a polypropylene homopolymer or a polypropylene copolymer.

3. , A bag according to claim 2, **characterised in that** the matrix-polymer is a polypropylene copolymer, preferably a polypropylene-random-copolymer.

4. A bag according to claim 1, **characterised in that** the phase polymer of the matrix-phase-polymer-system is a styrene-ethylene/butylene-styrene-triblock-polymer (SEBS), a styrene-ethylene/butylene-styrene-triblock-polymer (SEBS) with a styrene-ethylene/butylene-diblock fraction (SEB), a styrene-ethylene/propylene-styrene-triblock-polymer (SEPS), a styrene-butadiene-styrene-triblock-polymer (SBS) and/or a styrene-isoprene-styrene-triblock-polymer (SIS) and/or an ethylene-α-olefin-copolymer.

5. A bag according to one of claims 1 to 4, **characterised in that** the fraction of phase polymer in the polymer-system is in the range from 1 to 40, preferably 10 to 20% by weight, in relation to the matrix-phase-polymer-system.

6. A bag according to one of claims 1 to 4, **characterised in that** the film is multilayer and coextruded.

7. A method of producing an unfilled heat sterilised emptying medical bag according to claims 1 to 6, **characterised in that** the welding is carried out in the outer marginal portion (14, 16) at a temperature which allows no tearing open of the welded area without the bag being destroyed.

## Revendications

1. Sac vide à usage médical (10) exempt de matières de charge et qui a été stérilisé à la chaleur, comprenant au moins une chambre (12) et au moins un raccord tubulaire (18), dont la zone marginale (14, 16) a été soudée sur tout le pourtour, que l'on obtient du sac vide par stérilisation à la chaleur, **caractérisé en ce que** la surface interne de la feuille est constituée d'un système de polymères de matrice - de phases, dans lequel les surfaces internes du sac n'adhèrent pas l'une à l'autre, sans avoir soumis les surfaces internes à un prétraitement mécanique ou sans les avoir munies d'endroits profilés.

2. Sac selon la revendication 1, **caractérisé en ce que** le polymère de matrice du système de polymères de matrice - de phases est un homopolymère de polyéthylène, homopolymère de polypropylène ou un copolymère de polypropylène.

3. Sac selon la revendication 1, **caractérisé en ce que** le polymère de matrice est un copolymère de polypropylène, de préférence un copolymère statistique de polypropylène.

4. Sac selon la revendication 1, **caractérisé en ce que** le polymère de phases du système de polymères de matrice - de phases est un polymère triséquencé de styrène - éthylène/butylène - styrène (SEBS), un polymère triséquencé de styrène - éthylène/butylène - styrène (SEBS) avec une fraction biséquencée de styrène - éthylène/butylène (SEB), un polymère triséquencé de styrène - éthylène/propylène - styrène (SEPS), un polymère triséquencé de styrène - butadiène - styrène (SBS) et/ou un polymère triséquencé de styrène - isoprène - styrène (SIS) et/ou un copolymère d'éthylène - α-oléfine.

5. Sac selon l'une quelconque des revendications 1 à 4, **caractérisé en ce que** la fraction du polymère de phases dans le système de polymères est présente dans le domaine de 1 à 40, de préférence de 10 à 20 % en poids, rapportée au système de polymères de matrice - de phases.

6. Sac selon l'une quelconque des revendications 1 à 4, **caractérisé en ce que** la feuille est une feuille multicouche et a été coextrudée.

7. Procédé pour la fabrication d'un sac vide à usage médical exempt de matières de charge et qui a été stérilisé à la chaleur selon les revendications 1 à 6, **caractérisé en ce que** le soudage dans la zone marginale externe (14, 16) est réalisé à une température qui ne permet pas la rupture de la zone soudée sans détruire le sac.
